# FASCICULE DE BREVET EUROPEEN

(11) **EP 2 384 736 B1**
(45) Date de publication et mention de la délivrance du brevet: **11.04.2018**
(21) Numéro de dépôt: 10171559.7
(22) Date de dépôt: 02.08.2010
(51) Int. Cl.: A61K 8/04, A61Q 5/00, A61Q 7/00

(54) **PROCÉDÉ DE TRAITEMENT COSMÉTIQUE DU CUIR CHEVELU**
VERFAHREN ZUR KOSMETISCHEN KOPFHAUTBEHANDLUNG
COSMETIC SCALP TREATMENT PROCESS

(30) Priorité: 13.08.2009 FR 0955671
(43) Date de publication de la demande: 09.11.2011
(73) Titulaire: L'Oréal, 75008 Paris (FR)
(72) Inventeur: De Noray, Ségolène, 75116 Paris (FR); Vic, Gabin, 45400, Semoy (FR); Parris, Eric, 95110 Sannois (FR)
(74) Mandataire: Nony

(56) Documents cités:
- DE-A1- 4 242 009
- DE-U1- 20 117 052
- US-A- 5 080 116
- US-A1- 2005 063 916
- Anonyme: "Colani", , 29 juin 2007 (2007-06-29), XP002579502, Extrait de l'Internet: URL:http://web.archive.org/web/20070629005 939/http://www.harder-airbrush.de/colani_0 2.html [extrait le 2010-04-23]
- Anonyme: "Colani", , XP002579503, Extrait de l'Internet: URL:http://www.harder-airbrush.de/colani_0 2.html [extrait le 2010-04-23]

## Description

La présente invention concerne les procédés et systèmes pour le traitement du cuir chevelu humain.

Il est connu d'appliquer un produit sur le cuir chevelu en le versant directement sur le crâne, à l'aide d'une canule. Le produit est par exemple un produit assainissant, apaisant, antipelliculaire, antichute ou visant à stimuler la repousse des cheveux.

L'utilisation de dispositifs permettant de verser directement le produit sur le cuir chevelu n'est pas entièrement satisfaisante, car il est difficile de maîtriser précisément la quantité de produit versée, et il existe un risque de coulure qui peut entraîner une perte de produit ainsi qu'un écoulement dans les yeux.

Par ailleurs, la répartition du produit sur le cuir chevelu peut ne pas être aussi homogène que souhaitée, ce qui peut amener à devoir le répartir avec les doigts, et oblige au port de gants, ce qui peut s'avérer problématique en cas de sensibilité du cuir chevelu ou de fragilité des cheveux aux frottements.

Il est par ailleurs connu d'utiliser des aérographes (en anglais *air brush*) pour l'application de produits de maquillage sur la peau, qui pulvérisent le produit sous forme de fines gouttelettes.

La demande GB 2 287 480 A divulgue l'utilisation d'un aérographe pour appliquer un produit de neutralisation sur les cheveux dans le cas de la réalisation d'une permanente. Un aérographe fonctionnant avec de l'air comprimé pour appliquer un produit de repousse des cheveux est connu du document DE 201 17 052 U1. Il existe un besoin pour perfectionner encore les traitements du cuir chevelu.

L'invention a ainsi pour objet, selon un premier de ses aspects, un procédé de traitement cosmétique, i.e. non thérapeutique, du cuir chevelu humain, dans lequel un produit cosmétique et/ou un gaz est appliqué sur le cuir chevelu à l'aide d'un dispositif fonctionnant avec un gaz comprimé, le dispositif étant un aérographe, la taille moyenne des particules de produit pulvérisé étant comprise entre 10 et 35 microns. Ce produit est de préférence autre qu'un produit de coloration des cheveux ou de mise en forme de la chevelure ou de soin des cheveux.

Le produit peut être un produit de traitement du cuir chevelu, choisi notamment parmi les produits antipelliculaires, antichute ou repousse des cheveux, antiséborrhée, antitaches ou de stimulation ou de protection du cuir chevelu, ce produit pouvant être cosmétique ou dermatologique.

Le produit est pulvérisé sous forme de gouttelettes et non sous forme de mousse.

Le produit peut être pulvérisé sur des zones du crane dépourvues de cheveux.

De préférence, ce produit n'est pas une mousse.

De préférence le gaz comprimé est de l'air comprimé. Le gaz comprimé utilisé peut ainsi être non inflammable, non fluoré ou hydrocarboné, et facilement disponible.

De préférence le gaz comprimé utilisé est obtenu autrement qu'à partir d'un gaz liquéfié. Le dispositif de pulvérisation est différent d'un flacon aérosol à gaz liquéfié.

Le produit à pulvériser peut être stocké à l'état non pressurisé (i.e. à pression atmosphérique) dans le dispositif. Selon l'invention, le dispositif de pulvérisation est un aérographe.

L'utilisation d'un aérographe procure de nombreux avantages.

Comparativement à une application par simple versement du produit sur le cuir chevelu, l'aérographe peut apporter une plus grande surface de contact, une sensation de massage et de fraicheur. De plus, l'application peut être faiblement mouillante et sans coulure. L'aérographe peut permettre d'éviter l'emploi de gants tout en bénéficiant d'une hygiène satisfaisante, de par l'absence de contact avec le cuir chevelu.

Par rapport à certains dispositifs autonomes de pulvérisation, l'utilisation d'un aérographe permet de modifier aisément les paramètres de fonctionnement pour changer les effets engendrés, en cours d'utilisation, au gré de l'utilisateur ; ce dernier peut, par exemple, ajuster le débit du jet de produit en fonction du traitement à réaliser. De plus, le volume du gaz (de préférence l'air) qui sert de vecteur est illimité lorsque l'aérographe est relié à un compresseur. Cet air est de préférence non enrichi en oxygène. Le produit à pulvériser peut être renouvelé facilement et l'on peut aisément utiliser successivement avec un même aérographe des produits différents. Enfin, l'utilisateur peut sélectivement envoyer le gaz vecteur (de préférence de l'air) ou un mélange de gaz vecteur et de produit, si l'aérographe le permet ; l'envoi d'air peut être utile pour stimuler la zone avant et/ou après traitement et/ou accélérer le séchage du produit.

Le mélange entre le gaz vecteur et le produit à pulvériser peut se faire à l'intérieur de l'aérographe, ou à l'extérieur.

Dans des variantes de mise en oeuvre, l'aérographe peut éclairer la zone traitée pour aider l'utilisateur dans son geste, par exemple générer un point lumineux matérialisant l'axe de projection du produit. Cette aide peut s'avérer utile lorsque le produit pulvérisé n'est pas coloré et que les cheveux sont mouillés, par exemple. L'aérographe peut être utilisé pour peigner les cheveux, et peut comporter à cet effet un embout coiffant, situé par exemple du côté opposé à celui par lequel le produit est distribué ou, de préférence, dans le prolongement d'une poignée servant à manipuler l'aérographe, notamment lorsque ce dernier est de type pistolet.

Le produit cosmétique pulvérisé peut être choisi parmi les produits antipelliculaires, antichute, de repousse des cheveux ou de stimulation ou de protection du cuir chevelu, entre autres. Par « produit cosmétique », il faut comprendre un produit qui n'a pas d'action thérapeutique et qui peut être appliqué sans contrôle médical.

L'application peut s'effectuer sans contact de l'aérographe avec le cuir chevelu, sauf éventuellement l'embout de peignage, lequel est par exemple amovible, pour permettre son remplacement après nettoyage ou son choix parmi plusieurs embouts de peignage ayant des caractéristiques différentes, par exemple relativement au nombre de dents ou à leur disposition.

L'application du produit peut s'effectuer sur cheveux secs ou mouillés. L'application peut s'effectuer après un shampoing, par exemple.

Le gaz vecteur, notamment l'air, peut éventuellement être réchauffé ou refroidi avant d'atteindre la buse de sortie de l'aérographe. Un réchauffement du gaz vecteur peut accélérer le séchage du produit pulvérisé ou favoriser l'action d'un actif, par exemple en activant la circulation sanguine locale. Un refroidissement peut procurer une sensation de fraîcheur additionnelle.

Dans tout ce qui suit, lorsque des intervalles de valeurs sont mentionnés, les bornes de l'intervalle sont incluses. L'aérographe peut être maintenu à une distance comprise entre 1 et 10 cm du cuir chevelu, de préférence 1 à 5 cm, lors de la pulvérisation du produit.

La pulvérisation du produit peut s'effectuer après avoir formé une raie dans les cheveux. Pour former une telle raie, l'utilisateur peut se servir d'un peigne ou de l'aérographe notamment lorsque celui-ci est muni d'un embout de peignage. Le procédé selon l'invention peut comporter, dans un exemple de mise en oeuvre, l'application sur le cuir chevelu et/ou les cheveux d'un premier produit, et l'application sur le cuir chevelu à l'aide de l'aérographe d'un deuxième produit, le choix de ce deuxième produit s'effectuant en fonction du premier ou inversement. Le deuxième produit peut être appliqué avant le premier ou inversement, selon le traitement à réaliser.

Le premier produit, lorsqu'appliqué préalablement au deuxième, peut faciliter l'action de ce dernier. Les deux produits peuvent encore réagir entre eux, le cas échéant.

L'invention a encore pour objet, selon un autre de ses aspects, un système de traitement du cuir chevelu humain tel que défini dans la revendication 8. Le dispositif est un aérographe et le gaz comprimé est de préférence de l'air comprimé.

Le produit peut contenir au moins l'un des constituants des formules exemplifiées plus loin, par exemple la formule C', notamment tous leurs constituants actifs.

Par "aérographe comportant un réservoir" il faut comprendre que le corps de l'aérographe porte le réservoir ou qu'il est relié à celui-ci par un flexible. L'aérographe comporte une buse de sortie, de diamètre compris par exemple entre 0,1 et 1 mm, mieux 0,2 et 0,7 mm. La pression relative de l'air peut être comprise entre 0,2 et 3 bar, mieux 0,4 et 1 bar, en entrée de l'aérographe. La taille moyenne (c'est-à-dire le diamètre moyen volumique des particules dit D_{[4,3]}) des particules de produit pulvérisées est comprise entre 10 et 35 µm. Le débit de produit peut être inférieur ou égal à 10 g/min. L'aérographe peut comporter un embout de peignage. L'aérographe peut comporter une poignée, notamment en étant de type pistolet, et l'embout de peignage peut être situé dans le prolongement de la poignée. L'embout de peignage peut se fixer de façon amovible sur l'aérographe, notamment sur la poignée.

Le gaz vecteur, notamment l'air, peut être chauffé ou refroidi avant sa sortie de l'aérographe, par exemple en sortie d'un compresseur ou régulateur. La température de sortie de l'aérographe est par exemple comprise entre 5 et 60 °C.

Le spray émis par l'aérographe peut être de section circulaire ou autre. L'aérographe peut comporter un organe de commande de l'alimentation en produit, afin de permettre à l'opérateur de pulvériser sélectivement le produit. Cet organe de commande comporte par exemple une surface sur laquelle un doigt de l'utilisateur appuie. L'aérographe peut comporter une source lumineuse pour éclairer le cuir chevelu, par exemple un laser émettant dans le rouge. Cette source lumineuse peut émettre sensiblement dans l'axe de pulvérisation, pour éclairer la surface traitée, et peut aider à orienter l'aérographe.

La source lumineuse peut être alimentée par au moins une pile ou accumulateur porté par l'aérographe. En variante, le flexible d'alimentation peut comporter des conducteurs électriques. L'allumage de la source peut être lié à la pulvérisation ou non du produit. Par exemple, l'organe de commande qui ouvre l'alimentation en produit ou déclenche l'arrivée de gaz vecteur, peut agir également sur le fonctionnement de la source lumineuse.

### Actifs

Comme mentionné ci-dessus, le réservoir de l'aérographe comprend un produit de traitement du cuir chevelu, choisi notamment parmi les produits antipelliculaires, antichute ou repousse des cheveux, antiséborrhée, anti-inflammatoires, anti-irritations ou apaisant, antitaches ou de stimulation ou de protection du cuir chevelu, ce produit pouvant être cosmétique ou dermatologique.

Il peut s'agir d'un ou plusieurs actifs choisi parmi les suivants.

On entend par actif anti-séborrhéique un composé capable de réguler l'activité des glandes sébacées.

### Actifs anti-séborrhéiques

Un actif anti-séborrhéique convenant à l'invention peut, notamment, être choisi parmi l'acide rétinoïque, le peroxyde de benzoyle, le soufre, la vitamine B6 (ou pyridoxine), le chlorure de sélénium, la criste marine ; les mélanges d'extrait de cannelle, de thé et d'octanoylglycine tel que le Sepicontrol A5 TEA® de chez Seppic ; le mélange de cannelle, de sarcosine et d'octanoylglycine, commercialisé notamment par la société SEPPIC sous la dénomination commerciale Sepicontrol A5® ; les sels de zinc tels que le gluconate de zinc, le pyrrolidone carboxylate de zinc (ou pidolate de zinc), le lactate de zinc, l'aspartate de zinc, le carboxylate de zinc, le salicylate de zinc, le cystéate de zinc ; les dérivés de cuivre et en particulier le pidolate de cuivre tel que Cuivridone® de Solabia ; des extraits de végétaux des espèces *Arnica montana, Cinchona succirubra, Eugenia caryophyllata, Humulus lupulus, Hypericum perforatum, Mentha piperita, Rosmarinus afficinalis, Salvia oficinalis* et *Thymus vulgaris,* tous commercialisés par exemple par la société MARUZEN ; les extraits de reine des prés (*spiraea ulamaria*) tel que celui vendu sous la dénomination Sébonormine® par la société Silab ; les extraits d'algue *Laminaria saccharina* tel que celui vendu sous la dénomination Phlorogine® par la société Biotechmarine ; les mélanges d'extraits de racines de pimprenelle (*Sanguisorba officinalis*/*Poterium officinale*), de rhizomes de gingembre (*Zingiber officinalis*) et d'écorce de cannelier (*Cinnamomum cassia*) tel que celui vendu sous la dénomination Sebustop® par la société Solabia ; les extraits de graines de lin tel que celui vendu sous la dénomination Linumine® par la société Lucas Meyer ; les extraits de Phellodendron tels que ceux vendu sous la dénomination Phellodendron extract BG par la société Maruzen ou Oubaku liquid B par la société Ichimaru Pharcos ; les mélanges d'huile d'argan, d'extrait de *Serenoa serrulata* (saw palmetto) et d'extrait de graines de sésame tel que celui vendu sous la dénomination Regu SEB® par la société Pentapharm ; les mélanges d'extraits d'epilobe, de *Terminalia chebula,* de capucine et de zinc biodisponible (microalgues) tel que celui vendu sous la dénomination Seborilys® par la société Green Tech ; les extraits de *Pygeum afrianum* tel que celui vendu sous la dénomination Pygeum afrianum sterolic lipid extract par la société Euromed ; les extraits de *Serenoa serrulata* tels que ceux vendus sous les dénomination Viapure Sabal par la société Actives International, ou ceux vendus par la société Euromed ; les mélanges d'extraits de plantain, de *Berberis aquifolium* et de salicylate de sodium tels que celui vendu sous la dénomination Seboclear® par la société Rahn ; l'extrait de clou de girofle tel que celui vendu sous la dénomination Clove extract Powder par la société Maruzen ; l'huile d'argan telle que celle vendue sous la dénomination Lipofructyl® par les Laboratoires Sérobiologiques ; les filtrats de protéine lactique tels que celui vendu sous la dénomination Normaseb® par la société Sederma ; les extraits d'algue *Laminaria,* tel que celui vendu sous la dénomination Laminarghane® par la société Biotechmarine ; les oligosaccharides d'algue *Laminaria digitata* tel que celui vendu sous la dénomination Phycosaccharide AC par la société Codif ; les extraits de sucre de canne tel que celui commercialisé sous la dénomination Palicosanol® par la société Sabinsa ; l'huile de schiste sulfonée, telle que celle vendue sous la dénomination Ichtyol Pale® par la société Ichthyol ; les extraits d'ulmaire (*spiraea ulmaria*) tels que celui vendu sous la dénomination Cytobiol® Ulmaire par la société Libiol ; l'acide sébacique, notamment vendu sous la forme d'un gel de polyacrylate de sodium sous la dénomination Sebosoft® par la société Sederma ; les glucomannanes extraits de tubercule de konjac et modifié par des chaînes alkylsulfonates tel que celui vendu sous la dénomination Biopol Beta par la société Arch Chemical ; les extraits de *Sophora angustifolia,* tels que ceux vendus sous la dénomination Sophora powder ou Sophora extract par la société Bioland ; les extraits de *Cinchona succirubra* bark tel que celui vendu sous la dénomination le Red bark HS par la société Alban Muller ; les extraits de *Quillaja saponaria* tel que celui vendu sous la dénomination Panama wood HS par la société Alban Muller ; la glycine greffée sur chaîne undécylénique ou sur une chaîne octanoyle , telles que celles vendues sous les dénominations Lipacide UG OR, Lipacide C₈G par la société Seppic ; le mélange d'acide oléanolique et d'acide nordihydroguaïarëtique, tel que celui vendus sous la forme d'un gel sous la dénomination AC.Net par la société Sederma ; l'acide phthalimidoperoxyhexanoïque ; le citrate de trialkyle(C₁₂-C₁₃) vendu sous la dénomination COSMACOL® ECI par la société Sasol ; le citrate de trialkyle(C₁₄-C₁₅) vendu sous la dénomination COSMACOL® ECL par la société Sasol ; l'acide 10-hydroxydécanoïque, et notamment les mélanges d'acide 10-hydroxydécanoïque, d'acide sébacique et de 1,10-décandiol tels que celui vendu sous la dénomination Acnacidol® BG par la société Vincience ; les hydrolysats de protéines de levure tels que l'ASEBIOL LS de COGNIS (mélange avec vitamines) ; et leurs mélanges.

### Actifs anti-pelliculaires

On entend par actif anti-pelliculaire un composé capable de prévenir l'apparition des pellicules, diminuer leur nombre et/ou les faire disparaître totalement.

Un actif anti-pelliculaire convenant à l'invention peut, notamment, être choisi parmi :
- les dérivés de 1-hydroxy-2-pyridone tels que le 1-hydroxy-4-méthyl-2-pyridone, 1-hydroxy-6-méthylpyridone, le 1-hydroxy-4,6-diméthyl-2-pyridone, le 1-hydroxy-4-méthyl-6-(2,4,4-triméthylpentyl)-2-pyridone, le 1 "hydroxy-4-méthyl-6-cyclohexyl-2-pyridone, le 1-hydroxy-4-méthyl-6-(méthyl-cyclohexyl)2-pyridone, le 1-hydroxy-4-méthyl-6-(2-bicyclo[2,2,1]heptyl)-2-pyridone, le 1 -hydroxy-4-méthyl-6(4-méthylphenyl)-2-pyridone, le 1-hydroxy-4-méthyl-6[1-[4-nitrophenoxy]-butyl]-2-pyridone, le 1-hydroxy-4-méthyl-6-(4-cyanaphénoxyméthyl-2-pyridone), le 1-hydroxy-4-méthyl-6-(phenylsulfonylméthyl)-2-pyridone, le 1-hydroxy-4-méthyl-6-(4-bromobenzyl)-2-pyridone et leurs sels ; A titre de dérivé 1-hydroxy-2-pyridone préféré, on peut citer le produit commercialisé par la société HOECHST sous la dénomination octopyrox (1-hydroxy-4-méthyl-6-(2,4,4-triméthylpenthyl)-2-pyridone, sel de monoéthanolamine.
- les sels de pyridinethione notamment les sels de calcium, de magnésium, de barium, de strontium, de zinc, de cadmium, d'étain et de zirconium. Le sel de zinc de pyridinethione est particulièrement préféré. Le sel de zinc de pyridinethione est notamment commercialisé sous la dénomination Omadine de zinc par la société OLIN.
- les trihalogéno carbamide de formule: 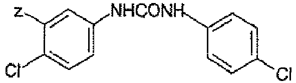 dans laquelle représente un atome d'halogène comme le chlore ou un groupement trihalogénoalkyle en C₁-C₄ tel que CF₃.
- le triclosan représenté par la formule : 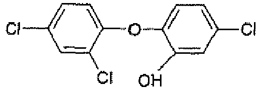
- les composés azolés tels que le climbazole, le kétoconazole, le clotrinazole, l'econazole, l'isoconazole et le miconazole.
- les polymères antifongiques tels que l'amphotéricine B ou la nystatine.
- les sulfures de sélénium en particulier ceux de formule Sₓ Se ₈₋ₓ, x allant de 1 à 7.
- le soufre sous ses différentes formes, le sulfure de cadmium, l'allantoïne, les goudrons de houille ou de bois et leurs dérivés en particulier l'huile de cade, l'acide salicylique, l'acide undécylénique, l'acide fumarique, les allylamines telle que la terbinafine.
- l'acide ellagique,
- le disufure de sélénium.

### Composés favorisant la croissance des fibres kératiniques humaines et/ou limitant leur chute et/ou l'augmentation de leur densité.

Ces composés additionnels sont notamment choisis parmi les inhibiteurs de lipoxygénase tels que décrits dans EP 648488, les inhibiteurs de bradykinine décrits notamment dans EP 845700, les prostaglandines et leurs dérivés notamment ceux décrits dans WO 98/33497, WO 95/11003, JP 97-100091, JP 96-134242, les agonistes ou antagonistes des récepteurs des prostaglandines, les analogues non prostanoïques de prostaglandines tels que décrits dans EP 1175891 et EP1175890, WO 01/74307, WO 01/74313, WO 01/74314, WO 01/74315 ou WO 01/72268, leurs mélanges.

Comme autres composés actifs additionnels favorisant la pousse des fibres kératiniques et/ou limitant leur chute (notamment les cheveux ou les cils) pouvant être présents dans la composition selon l'invention on peut citer les vasodilatateurs, les antiandrogènes, les cyclosporines et leurs analogues, les antimicrobiens et antifongiques, les anti-inflammatoires, les rétinoïdes, seuls ou en mélange.

Les vasodilatateurs utilisables sont notamment les agonistes des canaux potassium incluant le minoxidil ainsi que les composés décrits dans les brevets US 3 382247, 5 756092, 5 772990, 5 760043, 5 466694, 5 438058, 4 973474, la cromakalim, le nicorandil et le diaxozide, seuls ou en association.

Les esters d'acide nicotinique, dont notamment le nicotinate de tocophérol, le nicotinate de benzyle et les nicotinates d'alkyles en C1-C6 comme les nicotinates de méthyle ou d'hexyle peuvent être utilisés comme vasodilatateurs.

Les anti-androgènes utilisables incluent notamment les inhibiteurs stéroïdiens ou non stéroïdiens de 5α-réductase, comme le finastéride et les composés décrits dans US 5 516779, l'acétate de cyprostérone, l'acide azélaïque, ses sels et ses dérivés et les composés décrits dans US 5 480913, le flutamide, l'oxendolone, la spironolactone, le diéthylstilbestrol et les composés décrits dans les brevets US 5 411981, 5 565467 et 4910226.

Les anti-inflammatoires peuvent être choisis parmi les anti-inflammatoires stéroïdiens comme les glucocorticoïdes, les corticostéroïdes (par exemple : l'hydrocortisone) et les anti-inflammatoires non stéroïdiens comme l'acide glycyrrhétinique et l'α-bisabolol, la benzydamine, l'acide salicylique et les composés décrits dans EP 0770399, WO 94/06434 et FR 2 268523.

Les rétinoïdes peuvent être choisis parmi l'acide rétinoïque l'isotrétinoïne, l'acitrétine, le tazarotène, le rétinal et l'adapalène.

Comme autres composés actifs pour favoriser la pousse et/ou limiter la chute des fibres kératiniques comme les cheveux et les cils, on peut citer l'aminexil, le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, l'arginine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les benzophénones et l'hydantoïne, les vitamines comme la vitamine D, les analogues de la vitamine B12, le panthénol ; la vitamine B8, les triterpènes comme l'acide ursolique et les composés décrits dans US 5529769, US 5468888, US 5631282 ; les agents antiprurigineux comme la thénaldine, la triméprazine ou la cyproheptadine ; les antiparasitaires, en particulier le métronidazole, le crotamiton ou les pyréthrinoïdes ; les agents antagonistes de calcium, comme la cinnarizine, le diltiazem, la nimodipine, vérapamil, l'alvérine et la nifédipine ; les hormones telles que l'estriol ou ses analogues, la thyroxine et ses sels, la progestérone ; les agonistes du récepteur FP (récepteur aux prostaglandines du type F) tels que le latanoprost, l'acide (5E)-7-{(1R,2R,3R,5S)-3,5-dihydroxy-2-[(3R)-3-hydroxy-5-phényl pentyl]cyclopentyl}hept-5-énoique, le bimatoprost, le travoprost, l'unoprostone et le butaprost ; les dérivés O-acylés obtenus par estérification, partielle ou totale, de la vitamine F par le glucose tels que décrits dans la demande EP1688128 ; les inhibiteurs de la 15-hydroxy prostaglandine déshydrogénase ; leurs mélanges.

Comme autres composés actifs pour favoriser la pousse et/ou limiter la chute des fibres kératiniques comme les cheveux et les cils, utilisables en association avec le composé de formule (I), on peut citer les dérivés pyridine-dicarboxylate ou de l'un de leurs sels tels que ceux décrits dans la demande EP1352629 et plus particulièrement le pyridine-2,4-dicarboxylate de diéthyle.

Comme actif anti-irritation/apaisant, outre les antiinflammatoires déjà évoqués, on peut citer les sels de strontium, l'acide β-glyccyrrhétinique, l'acide glyccyrhizique, l'azulène, les huiles essentielles, les extraits de camomille, d'avoine, d'aloe vera, de verveine, de tilleul et de réglisse.

L'invention pourra être mieux comprise à la lecture de la description détaillée qui va suivre, d'exemples non limitatifs de mise en oeuvre de celle-ci, et à l'examen du dessin annexé, sur lequel :
- la figure 1 représente de façon schématique et partielle un exemple de système de traitement pouvant être utilisé selon l'invention, et
- la figure 2 représente un exemple d'aérographe.

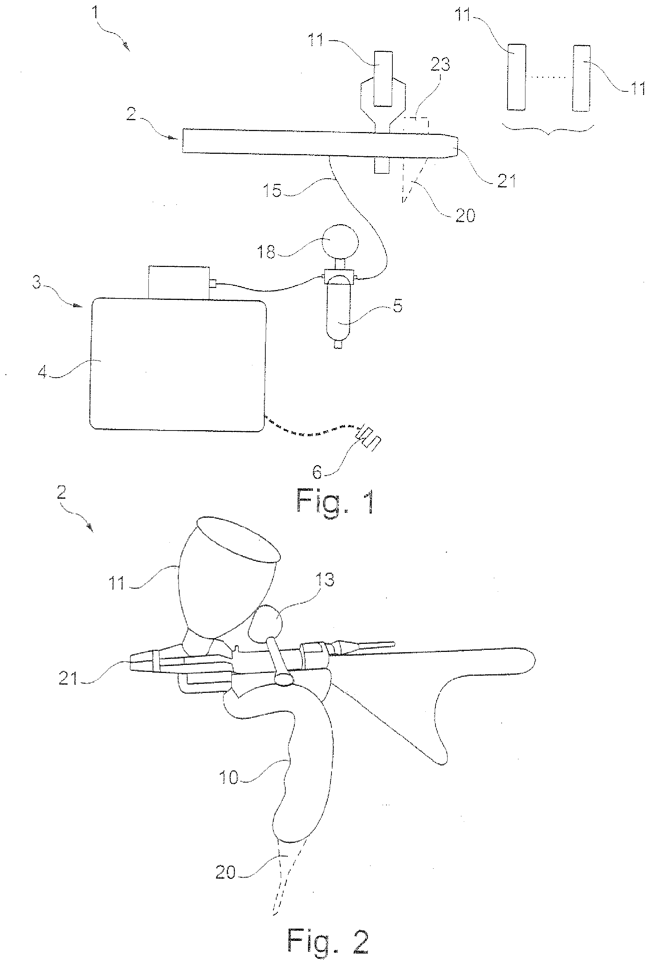

On a représenté à la figure 1 un exemple de système de traitement 1 pouvant être utilisé pour mettre en oeuvre le procédé de traitement selon l'invention.

Le système de traitement 1 comporte, comme illustré, un aérographe 2 relié à une source de gaz comprimé, comportant par exemple un compresseur à air 4 relié à l'aérographe 2 par l'intermédiaire d'un régulateur de pression 5, de façon connue en soi, et d'un flexible 15. La source de gaz comprimé peut être également une capsule interchangeable ou rechargeable de gaz comprimé.

Le fonctionnement du compresseur 4 peut éventuellement être commandé par un système 6 actionnable au pied ou par tout autre moyen de commande, par exemple être commandable manuellement ou par la voix. Le fonctionnement du compresseur peut encore être déclenché automatiquement en détectant un mouvement de l'aérographe ou son départ d'une station d'accueil, dans des variantes.

Le compresseur 4 présente de préférence une émission sonore inférieure à 40 dB et fournit de préférence un débit d'air supérieur ou égal à 15 l/min, le compresseur pouvant être avec ou sans réserve d'air, de préférence avec protection thermique et de préférence encore ayant une sortie équipée d'un raccord rapide pour la fixation du flexible de raccordement à l'aérographe.

Le compresseur 4 peut être à simple piston, à double piston, à sec ou à bain d'huile, et le régulateur de pression 5 est de préférence équipé d'un manomètre 18.

L'aérographe 2 comporte de façon connue en soi une partie de préhension qui est par exemple définie par le corps de l'aérographe lorsque celui-ci présente une forme de stylo ou par une poignée 10 lorsque celui-ci est de type pistolet, comme illustré sur la figure 2.

L'aérographe 2 peut porter un réservoir 11 contenant le produit à pulvériser, par exemple sous la forme d'un godet amovible, éventuellement rechargeable, qui peut se situer en partie supérieure de l'aérographe de façon à permettre un écoulement par gravité du produit contenu à l'intérieur dans un canal de distribution où le produit est mélangé à un gaz vecteur avant d'être pulvérisé.

Le réservoir 11 peut être muni de graduations afin de permettre à l'utilisateur de visualiser plus facilement la quantité de produit disponible. Le volume de produit contenu dans le réservoir 11 est par exemple compris entre 1 et 100 ml.

L'aérographe 2 peut comporter tout moyen de déclenchement de la pulvérisation, par exemple sous la forme d'un organe de commande tel qu'une manette 13, comme illustré à la figure 2, pouvant être actionnée par un doigt de la main de l'utilisateur qui tient l'aérographe.

A titre d'exemples d'aérographes pouvant être utilisés dans l'invention, on peut citer celui de référence A 4700 de la société Azteck, celui de référence Kustom micron CM de la société Iwata, celui de référence Evolution infiniti 2 in 1 de la société Harder et Steenbeck, ces aérographes étant de type à prise stylo, avec réservoir sous forme de godet.

On peut encore utiliser l'aérographe de référence Kustom revolution TR de la société Iwata, ou celui de référence Colani® de la société Harder et Steenbeck, ce dernier modèle étant préféré pour sa prise en main qui convient tout particulièrement au traitement du cuir chevelu, et étant celui représenté à la figure 2.

De préférence, le flexible 15 qui relie l'aérographe au compresseur et notamment au régulateur d'air 5 présente une longueur inférieure ou égale à 5 m et son diamètre intérieur est par exemple égal à 4 mm. Le flexible 15 est de préférence muni d'embouts à raccordement rapide.

L'aérographe 2 peut avantageusement comporter un embout de peignage 20 qui peut permettre à l'utilisateur qui tient l'aérographe d'écarter les cheveux au cours du traitement afin de dégager le cuir chevelu, notamment en formant une raie dans les cheveux. Cet embout de peignage 20 est par exemple situé dans le prolongement de la poignée 10 dans le cas d'un aérographe du type pistolet comme celui illustré à la figure 2. Il peut encore être fixé ailleurs, par exemple être situé au niveau de l'extrémité arrière de l'aérographe, c'est-à-dire celle opposée à la buse 21 de projection du produit, ou sur le côté ailleurs qu'au niveau d'une poignée, comme illustré schématiquement à la figure 1.

L'aérographe 2 peut éventuellement comporter un système d'éclairage 23 de la zone vers laquelle est projeté le produit. Ce système d'éclairage 23 comporte par exemple une ou plusieurs diodes électroluminescentes blanches ou d'une autre couleur. L'angle de divergence du faisceau lumineux émis par la source lumineuse 23 peut être choisi le cas échéant de façon à ce que l'étendue éclairée corresponde sensiblement à la surface touchée par la projection du produit à une distance prédéfinie d'utilisation.

La source lumineuse 23 peut encore comporter un pointeur laser permettant de projeter sur la zone traitée un point lumineux ou une mire facilitant l'orientation par l'utilisateur de l'aérographe dans la bonne direction. Cela peut permettre de diriger au mieux le produit sur le cuir chevelu en réduisant les pertes par pulvérisation sur les cheveux.

Le cas échéant, la mire projetée apparaît floue lorsque l'aérographe n'est pas à la bonne distance de pulvérisation.

La source lumineuse 23 peut encore viser à réaliser un traitement de luminothérapie sur le cuir chevelu, par exemple en émettant à une ou plusieurs longueurs d'onde choisies pour exercer un effet bénéfique sur la repousse des cheveux, notamment une longueur d'onde dans le rouge.

L'aérographe 2 est proposé à l'utilisateur avec plusieurs réservoirs additionnels 11, comme illustré à la figure 1, contenant par exemple le même produit que celui présent dans le réservoir en place sur l'aérographe, de façon à permettre à l'utilisateur de remplacer rapidement un réservoir vide par un réservoir plein. Les réservoirs additionnels 11 peuvent être munis d'un connecteur rapide, se fermant automatiquement quand le réservoir est retiré de l'aérographe et s'ouvrant quant il est mis en place sur l'aérographe. Les réservoirs 11 présentent un moyen d'obturation tel qu'un bouchon, perforé par un moyen d'ouverture, tel qu'une aiguille, présent sur l'aérographe. Les réservoirs additionnels peuvent encore comporter des produits différents, visant à réaliser des traitements différents, et l'utilisateur peut choisir entre les réservoirs celui qui correspond au traitement à effectuer.

Les paramètres de pulvérisation, notamment le débit du gaz vecteur, (de préférence l'air,) et/ou le débit de produit pulvérisé, peuvent être adaptés manuellement par l'utilisateur à chaque changement de réservoir 11, lorsque cela est nécessaire, ou au cours de l'utilisation.

Dans une variante, le système de traitement 1 est agencé pour adapter automatiquement les paramètres de fonctionnement en fonction du réservoir en place et du produit contenu dedans, par exemple grâce à une reconnaissance par le système de traitement, par exemple par l'aérographe 2, du réservoir utilisé. Les réservoirs contenant différents produits peuvent par exemple présenter des identifiants qui sont reconnus par le système de traitement. Par exemple, les différents réservoirs peuvent comporter une puce électronique, un code optique ou des reliefs qui sont détectés par un détecteur adapté, par exemple présent sur l'aérographe 2, un processeur permettant de commander au moins un actuateur pour modifier un paramètre de fonctionnement en fonction de l'information lue.

La pression d'air comprimé à l'arrivée dans l'aérographe 2 peut être comprise entre 0,2 et 3 bar, étant par exemple de l'ordre de 0,6 bar.

La buse 21 équipant l'aérographe est choisie de telle sorte que la taille moyenne des gouttelettes de produit pulvérisé soit centrée sur une valeur comprise entre 10 et 35 µm, par exemple de l'ordre de 23 µm (taille mesurée à une distance de 15 cm de la sortie de la buse).

Le choix du diamètre de la buse peut permettre d'agir sur la granulométrie du spray ainsi que sur sa divergence. On peut utiliser par exemple une buse de diamètre de 0,2 mm, notamment sur l'aérographe de référence Colani, ce qui produit un jet précis et ressenti par l'utilisateur. Un diamètre de buse de 0,2 mm convient tout particulièrement lorsque utilisé conjointement avec une pression de 0,8 bar en entrée de l'aérographe, pour obtenir un débit de produit de 3,5 g/min environ. Dans le cas de l'aérographe Kustom micron CM, le diamètre de buse est de 0,5 mm et la pression d'entrée est de 0,4 bar avec un débit de produit de l'ordre de 6g/mn. De préférence, on veille à ce que le taux de particules de taille inférieure à 10 µm soit inférieur à 20 % et celui de taille inférieure à 5 µm soit inférieur à 5 %.

Le système de traitement 1 peut être utilisé sur un cuir chevelu sec ou humide, pour pulvériser sur celui-ci des produits de traitement ayant notamment des propriétés antipelliculaires, antichute, de stimulation de la repousse des cheveux, antiséborrhée ou anti-inflammatoire, entre autres applications.

Dans des exemples de mise en oeuvre de l'invention, la pulvérisation du produit avec l'aérographe peut s'effectuer en combinaison avec l'application d'un traitement classique, par exemple un shampoing préalable au traitement par l'aérographe, un après-shampoing ou un masque de soin capillaire. Tous ces traitements conventionnels peuvent être suivis de la pulvérisation sur le cuir chevelu, sec ou humide, d'un produit de traitement du cuir chevelu, notamment antipelliculaire, antichute, de repousse, antiséborrhée ou anti-inflammatoire.

Dans d'autres exemples de mise en oeuvre de l'invention, on applique sur les cheveux et le cuir chevelu une huile stimulante ayant des propriétés d'anti-regraissage ainsi qu'une mousse lavante et l'on peut traiter ensuite le cuir chevelu par un produit antichute qui est pulvérisé avec l'aérographe, ce produit antichute étant choisi en fonction de la nature du produit appliqué précédemment.

L'utilisation de l'aérographe peut encore être suivie d'un traitement conventionnel tel qu'une permanente, un traitement défrisant, de décoloration, de décapage de couleur ou de coloration.

On peut par exemple appliquer à l'aide de l'aérographe un produit de protection du cuir chevelu, notamment anti-irritations/apaisant et/ou anti tâches, avant d'effectuer un traitement de permanente, de défrisage, de décoloration ou de décapage de couleur.

On donne dans la suite quelques exemples de formulation de produits pouvant être pulvérisés à l'aide de l'aérographe. Les proportions indiquées sont massiques.

Lors de l'utilisation de l'aérographe, l'opérateur peut tenir celui-ci d'une main et avec l'autre main s'aider d'un peigne pour dégager le cuir chevelu aux emplacements à traiter.

L'application du produit à l'aide de l'aérographe peut être suivie d'un massage, avant ou après la pulvérisation du produit. Ce massage peut être réalisé par exemple avec les doigts ou en s'aidant d'un dispositif de massage, amené au contact du cuir chevelu, par exemple vibrant.

Dans des exemples de mise en oeuvre, on peut appliquer un produit avec ou sans l'aérographe, puis souffler à l'aide de l'aérographe de l'air chaud sur la zone à traiter, par exemple pour activer un actif contenu dans le produit, par exemple un actif antichute. L'élévation de la température peut accélérer la circulation sanguine localement et favoriser l'action de l'actif. L'air chaud en sortie de l'aérographe peut ainsi être à une température comprise entre 30 et 60 °C.

Quel que soit le produit appliqué, de préférence, lors de l'utilisation de l'aérographe, on maintient la buse de sortie à une distance comprise entre 1 et 10 cm du cuir chevelu, par exemple 1 et 5 cm.

### Exemples

### Procédé de traitement seul

### Procédé de traitement combiné avec un traitement classique

| **Procédés** | **Traitements classiques** | **Produit pulvérisé avec l'aérographe** Cheveux et cuir chevelu secs ou humides |
|---|---|---|
| 2 | Shampooing | Formules de traitement du cuir chevelu : |
| 3 | Après shampooing | Anti pelliculaire |
| 4 | Shampooing + Après shampooing | Anti chute |
| | | Repousse |
| 5 | Masque de soin capillaire | Anti séborrhée |
| | | Anti inflammatoire,... |

| **Procédé** | **Traitement préalable** | **Produit pulvérisé avec l'aérographe** |
|---|---|---|
| 6 | Huile stimulante antiregraissage puis mousse lavante | Produit Anti chute formulé en fonction de la formulation du traitement préalable |

| **Procédés** | **Traitement par aérographe préalable** | **Traitement final** |
|---|---|---|
| 7 | Formules de protection du cuir chevelu : | Permanente |
| 8 | Anti irritations | Défrisant |
| 9 | Anti taches | Décoloration |
| 10 | | Décapage de couleur |

### Formule de repousse A :

| | **A** | **Procédés possibles** |
|---|---|---|
| Safflower glucoside | 3 | 1, 2, 3,4 et 5 |
| Ethanol | 50 | |
| Eau | 47 | |

### Formules antichute B et C :

| | **B** | **C** | **Procédés possibles** |
|---|---|---|---|
| Diaminopyrimidine oxide | 0,2 | 1,5 | 1, 2, 3, 4, 5 et 6 |
| Safflower glucoside | --- | 0.1 | |
| PEG-40 Hydrogenated castor oil | --- | 0.1 | |
| Ethanol | 48 | 50,7 | |
| Eau | 51,8 | 47.6 | |

### Formule antichute C' (pouvant être utilisée par exemple en substitution à la formule B ou C) :

| | |
|---|---|
| L-Arginine | 1,5 % |
| Pentothenate de calcium | 0,1 % |
| Amide nicotinique | 0,1 % |
| Acétate de DL alpha-tocophéryle | 0,05 % |
| PEG-40 huile de ricin hydrogénée | 1 % |
| L-Menthol | 0,1 % |
| Ethanol | 35 % |
| Eau | 62,15 % |

### Formules antipelliculaires D à I :

| | **D** | **E** | **F** | **G** | **H** | **I** | **Procédés possibles** |
|---|---|---|---|---|---|---|---|
| Piroctone Olamine (Octopirox) | 0,2 | --- | --- | --- | --- | --- | 1, 2, 3, 4 et 5 |
| Zinc pyrithione | 0.2 | --- | --- | --- | --- | --- | |
| Sulfure de Selenium | --- | 0.5 | --- | --- | --- | --- | |
| Acide Ellagique | --- | --- | 0.5 | --- | --- | --- | |
| parfum | 0.2 | --- | --- | --- | --- | --- | |
| Ethanol | 40,8 | 41,5 | 18,5 | --- | --- | 36,1 | |
| Eau | 58,8 | 58 | 81 | 80 | 80 | 62,9 | |

### Formules antiséborrhée H, I :

| | **H** | **I** | **Procédés possibles** |
|---|---|---|---|
| Zinc Pyrrolidone carboxylate | 1 | --- | 1, 2, 3, 4, et 5 |
| Hydrolyze yeast protein (ASEBIOL LS) | --- | 0.5 | |
| Ammonium glyccyhizate | --- | 0.2 | |
| Ethanol | --- | 40 | |
| Eau | 99 | 59,3 | |

### Procédé de traitement en utilisant la formule antichute C pour la mise en oeuvre du procédé 6

| | |
|---|---|
| 1. | traiter le cuir chevelu avec une huile stimulante (par exemple formule J). |
| 2. | mouiller à l'eau |
| 3. | traiter avec une mousse (par exemple la formule K) |
| 4. | rincer |
| 5. | sécher ou non (sèche cheveux) |
| 6. | appliquer 6 ml de la formule C antichute avec l'aérographe (par exemple Colani ®). Régler la pression d'air comprimé entre 0,5 et 0.8 bar en fonction de l'effet de massage désiré. Buse 0,2 mm et débit max du jet : 3,4 g/min |
| 7. | La solution appliquée, ne pas rincer et coiffer. |

### Formule mousse K

| | |
|---|---|
| Huile de ricin hydrogénée à 60 OE | 0,25 |
| Permulen TR2 | 0,15 |
| Glycérine | 4,0 |
| Ethanol | 13,0 |
| PEG 8 | 0 ou 0,10% |
| Gomme de xanthane | 0,2 |
| Acide lactique | 0,2 |
| Triéthanolamine | 0,6 |
| Diméthyléther | 20,0 |
| Mélange isobutene/propane/butane | 15,0 |
| Conservateurs | qs |
| Eau | qsp 100 g |

### Huile stimulante J

| | |
|---|---|
| Noctanoyl glycine | 0,5 |
| Quaternium 80 | 0,4 MA |
| Polysorbate 80 | 1,5 |
| Chlorure de céthyl méthyl ammonium | 0,5 |
| Merquat 100 | 1 MA |
| Glycérine | 1 |
| Hydroxyéthyl cellulose | 0,4 g |
| Conservateur | qs |
| Eau | qsp 100 g |

### Exemple comparatif

On verse fa formule antichute directement sur le cuir chevelu à partir d'un flacon muni d'une tétine, à partir de l'étape 6 de l'exemple précédent.

| Soin antichute application traditionnelle | Soin antichute appliqué avec l'aérographe (par exemple Colani ®) |
|---|---|
| Application en versant ou en pressant une tétine. | Application en tirant (ou appuyant) sur la gâchette d'arrivée de produit. |
| | ▪ Il est possible de déplacer l'outil à la surface du crane sans arrêter le jet. |
| ▪ Il faut régulièrement stopper pour éviter de détremper une même zone | |
| ▪ Difficile de maintenir cette action tout en déplaçant le flux à la surface du crâne (raie par raie) | ▪ Les coulures sont évitées. |
| | ▪ Application extrêmement précise raie par raie |
| ▪ La zone d'application du produit n'est pas précise | ▪ La zone traitée est quasi sèche, il est possible d'appliquer à nouveau si besoin. |
| ▪ Massage aux doigts pour s'assurer de la répartition de la formule. | |
| | ▪ Il n'est pas nécessaire de répartir le produit aux doigts. |

Bien entendu, l'invention n'est pas limitée aux exemples qui viennent d'être décrits.

On peut notamment utiliser le système de traitement alors que l'aérographe n'émet que le gaz vecteur, notamment de l'air, afin par exemple de sécher le produit précédemment appliqué ou stimuler son action.

On peut utiliser un même compresseur pour plusieurs aérographes, ce compresseur étant par exemple situé hors de la salle dans laquelle s'effectue le traitement, de façon à réduire les nuisances sonores.

Un réservoir porté par l'aérographe peut être remplacé par un réservoir relié à l'aérographe par un conduit, le produit étant aspiré dans l'aérographe par la dépression créée par effet Venturi par la circulation de gaz vecteur dans l'aérographe. Une pompe peut encore être utilisée pour amener le produit à pulvériser à l'aérographe.

De préférence, le spray pulvérisé est circulaire, mais des buses diverses peuvent être utilisées, de façon à avoir des sprays plats ou ayant une forme autre.

Le compresseur peut être remplacé par une réserve d'air comprimé, par exemple de l'air comprimé en bouteille.

L'expression "comportant un" doit être comprise comme étant synonyme de "comportant au moins un", sauf si le contraire est spécifié.

## Revendications

1. Procédé de traitement cosmétique, non thérapeutique, du cuir chevelu humain, dans lequel un produit cosmétique est appliqué sur le cuir chevelu à l'aide d'un dispositif de pulvérisation fonctionnant avec un gaz comprimé, le dispositif étant un aérographe,
la taille moyenne des particules de produit pulvérisé étant comprise entre 10 et 35 µm.

2. Procédé selon la revendication 1, dans lequel le gaz comprimé est de l'air comprimé.

3. Procédé selon les revendications 1 ou 2, dans lequel le gaz comprimé est produit par un compresseur ou dispensé à partir d'une capsule interchangeable ou rechargeable, le compresseur ou la capsule étant relié au dispositif de pulvérisation.

4. Procédé selon la revendication 4, l'application s'effectuant sans contact de l'aérographe (2) avec le cuir chevelu.

5. Procédé selon l'une quelconque des revendications 1 à 5, l'air étant réchauffé avant sa sortie du dispositif de pulvérisation, notamment de l'aérographe.

6. Procédé selon l'une quelconque des revendications 1 à 6, le produit étant pulvérisé avec un débit inférieur à 10 g/min.

7. Procédé selon l'une quelconque des revendications 1 à 7, le produit étant différent d'une mousse.

8. Système de traitement du cuir chevelu humain, comportant :
- un aérographe (2) comportant un réservoir (11) contenant un produit de traitement du cuir chevelu, choisi parmi les produits antipelliculaires, antichute, de repousse des cheveux, antiséborrhée, anti-inflammatoires, anti-irritations/apaisants, antitaches,
- plusieurs réservoirs additionnels, présentant un moyen d'obturation, contenant le même produit que celui présent dans le réservoir en place sur l'aérographe, l'aérographe étant agencé pour pulvériser ce produit avec une taille moyenne de particules comprise entre 10 et 35 µm.

9. Système selon la revendication précédente, l'aérographe comportant une buse (21) de sortie, de diamètre compris entre 0,1 et 1 mm, mieux 0,2 et 0,7 mm.

10. Système selon l'une des deux revendications immédiatement précédentes, la pression de l'air étant comprise entre 0,2 et 3 bar.

11. Système selon l'une quelconque des revendications 8 à 10, l'aérographe comportant un embout de peignage (20), notamment solidaire d'une poignée de l'aérographe, de préférence disposé à l'extrémité inférieure de la poignée, et/ou une source lumineuse (23) pour éclairer le cuir chevelu.

12. Système selon l'une quelconque des revendications 8 à 11, l'air étant chauffé ou refroidi avant sa sortie de l'aérographe.

13. Système selon l'une quelconque des revendications 8 à 12, le débit de produit en cours de pulvérisation étant inférieur à 10 g/min.

## Patentansprüche

1. Verfahren zur kosmetischen nicht therapeutischen Behandlung der menschlichen Kopfhaut, wobei ein kosmetisches Produkt auf die Kopfhaut mithilfe einer Sprühvorrichtung, die mit einem Druckgas arbeitet, aufgebracht wird, wobei die Vorrichtung eine Sprühpistole ist, wobei die mittlere Größe der Teilchen von versprühtem Produkt 10 bis 35 µm beträgt.

2. Verfahren nach Anspruch 1, wobei das Druckgas Druckluft ist.

3. Verfahren nach Anspruch 1 oder Anspruch 2, wobei das Druckgas durch einen Kompressor erzeugt oder aus einer austauschbaren oder wiederaufladbaren Druckflasche abgegeben wird, wobei der Kompressor oder die Druckflasche mit der Sprühvorrichtung verbunden ist.

4. Verfahren nach Anspruch 4, wobei das Aufbringen ohne Kontakt der Sprühpistole (2) mit der Kopfhaut erfolgt.

5. Verfahren nach einem der Ansprüche 1 bis 5, wobei die Luft vor ihrem Verlassen der Sprühvorrichtung, insbesondere der Sprühpistole, erwärmt wird.

6. Verfahren nach einem der Ansprüche 1 bis 6, wobei das Produkt mit einem Durchsatz von kleiner als 10 g/Min. versprüht wird.

7. Verfahren nach einem der Ansprüche 1 bis 7, wobei das Produkt von einem Schaum unterschiedlich ist.

8. System zur Behandlung der menschlichen Kopfhaut, folgendes umfassend:
- eine Sprühpistole (2), umfassend ein Reservoir (11), enthaltend ein Produkt zur Behandlung der Kopfhaut, das ausgewählt ist aus Produkten gegen Schuppen, Haarausfall, haarwuchsfördernden Produkten, Antiseborrhoe-Produkten, entzündungshemmenden Produkten, juckreizstillenden/-lindernden Produkten, schmutzabweisenden Produkten,
- wobei mehrere zusätzliche Reservoire, die ein Verschlussmittel aufweisen, das gleiche Produkt wie dasjenige enthalten, das in dem Reservoir in der Sprühpistole vorhanden ist, wobei die Sprühpistole zum Versprühen dieses Produkts mit einer mittleren Teilchengröße von 10 bis 35 µm ausgelegt ist.

9. Systeme nach dem vorangehenden Anspruch, wobei die Sprühpistole eine Austrittsdüse (21) mit einem Durchmesser von 0,1 bis 1 mm, bevorzugt von 0,2 bis 0,7 mm aufweist.

10. System nach einem der beiden unmittelbar vorangehenden Ansprüche, wobei der Druck der Luft 0,2 bis 3 bar beträgt.

11. System nach einem der Ansprüche 8 bis 10, wobei die Sprühpistole ein Kamm-Ende (20), insbesondere integral mit einem Handstück der Sprühpistole, das vorzugsweise am unteren Ende des Handstücks angeordnet ist, und/oder eine Lichtquelle (23) zum Beleuchten der Kopfhaut umfasst.

12. System nach einem der Ansprüche 8 bis 11, wobei die Luft vor ihrem Verlassen der Sprühpistole erwärmt oder abgekühlt wird.

13. System nach einem der Ansprüche 8 bis 12, wobei der Produkt-Durchsatz während des Sprühens kleiner als 10 g/Min. ist.

## Claims

1. A non-therapteutic cosmetic treatment method for treating the human scalp, wherein a cosmetic product is applied to the scalp by means of a spray device that operates with a compressed gas, the device being an airbrush,
the mean size of the sprayed particles of product lying in the range of 10 µm to 35 µm.

2. The method according to claim 1, wherein the compressed gas is compressed air.

3. The method according to claim 1 or 2, wherein the compressed gas is produced by a compressor or is dispensed from a capsule that is interchangeable or rechargeable, the compressor or the capsule being connected to the spray device.

4. The method according to claim 4, the application being performed without the airbrush (2) coming into contact with the scalp.

5. The method according to any one of claims 1 to 5, the air being heated before leaving the spray device, in particular the airbrush.

6. The method according to any one of claims 1 to 6, the product being sprayed with a flowrate that is less than 10 g/min.

7. The method according to any one of claims 1 to 7, the product being other than a foam.

8. A treatment system for treating the human scalp, comprising:
• an airbrush (2) including a reservoir (11) containing a product for treating the scalp, selected from: antidandruff, anti-hairloss, hair-regrowth, anti-seborrheic, anti-inflammatory, anti-irritant/soothing, and concealer compositions; and
• a plurality of additional reservoirs containing the same product as the product present in the reservoir that is in place on the airbrush, the airbrush being arranged to spray the product with a mean particle size lying in the range of 10 µm to 35 µm.

9. The system according to the preceding claim, the airbrush including an outlet nozzle (21) of diameter lying in the range of 0.1 mm to 1 mm, better in the range of 0.2 mm to 0.7 mm.

10. The system according to either one of the two immediately preceding claims, the pressure of the air lying in the range of 0.2 bars to 3 bars.

11. The system according to any one of claims 8 to 10, the airbrush including a comb endpiece (20), in particular secured to a handle of the airbrush, and/or a light source (23) for illuminating the scalp.

12. The system according to any one of claims 8 to 11, the air being heated or cooled before it leaves the airbrush.

13. The system according to any one of claims 8 to 12, the flowrate of composition during spraying being less than 10 g/min.
